# EUROPEAN PATENT APPLICATION

(11) **EP 3 508 564 A1**
(43) Date of publication of application: **10.07.2019**
(21) Application number: 17846161.2
(22) Date of filing: 18.08.2017
(51) Int. Cl.: C12M 3/00, C12M 1/00, C12N 5/071

(54) **CELL CULTURE CARRIER, CELL CULTURE CARRIER PREPARATION KIT, AND METHOD FOR PRODUCING GEL/CELL HYBRID TISSUE USING CELL CULTURE CARRIER AND CELL CULTURE CARRIER PREPARATION KIT**

(30) Priority: 31.08.2016 JP 2016169600
(71) Applicant: Osaka University, Suita-shi, Osaka 565-0871 (JP); Toray Industries, Inc., Tokyo 103-8666 (JP)
(72) Inventor: AKASHI, Mitsuru, Suita-shi Osaka 565-0871 (JP); KAKIZAWA, Yoshinori, Otsu-shi Shiga 520-8558 (JP)
(74) Representative: Kador & Partner PartG mbB
(86) International application number: PCT/JP2017/029608
(87) International publication number: WO 2018/043153

(57) **Abstract**

The present invention is to improve dynamic characteristics while maintaining temperature responsiveness in a cell culture support containing hydroxyalkyl chitosan, and to provide a cell culture support having temperature responsiveness, which contains temperature-responsive hydroxyalkyl chitosan and a water-soluble polymer selected from polyethylene glycol, derivatives thereof, hyaluronic acids, alginic acids and salts thereof.

## Description

### TECHNICAL FIELD

The present invention relates to a cell culture support, and more particularly to a temperature-responsive cell culture support containing a predetermined temperature-responsive polymer and a water-soluble polymer.

### BACKGROUND ART

A cell culture support is used for culturing a cellular tissue having a predetermined structure. For example, by culturing cells in a porous base material comprised of polylactic acid, the cells adhere to the base material, and a cellular tissue having a three-dimensional structure composed of the base material and the cells is obtained (for example, Non Patent Literature 1).

However, such base material was difficult to be removed after tissue formation, and it was difficult to obtain a cellular tissue with a high content of cells or composed of cells only.

In regenerative medicine, when transplanting a tissue composed of a base material and cells into the body, it has been a problem that the base material as an artifact remains or a very long period of time is required for the base material to disappear.

Therefore, using a stimuli-responsive material as a base material, it has been attempted to remove the base material by applying some kind of stimulus after tissue formation. As a stimuli-responsive material for such purpose, a temperature-responsive polymer whose state changes in response to temperature has attracted attention. For example, when a temperature-responsive polymer that becomes a gel (solid state) at a certain temperature or higher and a sol (liquid state) at a certain temperature or lower is used as a base material, the base material can be removed by solation by lowering temperature after formation of cellular tissue.

As the temperature-responsive polymer of which attention is focused, a polymer generally in a hydrated state showing a lower critical solution temperature (hereinafter abbreviated as "LCST") which dehydrates at a certain temperature or higher to change in volume, configuration, properties or the like, and a polymer showing an upper critical solution temperature (hereinafter abbreviated as "UCST") which hydrates at a certain temperature or higher to change in volume, configuration, properties or the like are known. As an example showing LCST, homopolymers or copolymers of N-isopropylacrylamide (NIPAM) and poloxamers are known. Particularly, poly N-isopropylacrylamide (PNIPAM) gels are extensively researched as medical materials and the like because volume transfer of swelling and contraction occurs at around 32°C close to body temperature (for example, Patent Literature 1).

However, since PNIPAM requires irreversible chemical crosslinking for gel formation, it is difficult to remove it after tissue formation. Poloxamer is not used because it shows toxicity, and also has problems of requiring high concentration for gelation and dissolving upon addition of medium.

On the other hand, hydroxyalkyl chitosan has an LCST around 25°C and is sol-gel-transferred, thus it can be removed after tissue formation, and it also has low toxicity, so it is expected to be utilized as a cell culture support (For example, Non Patent Literature 2 and Patent Literature 2).

### CITATION LIST

### PATENT LITERATURE

Patent Literature 1: JP 2006-223106 A
Patent Literature 2: WO 2015/129881 A

### NON-PATENT LITERATURE

Non Patent Literature 1: Nature Biotechnology, vol.12, 1994, p689
Non Patent Literature 2: Biomaterials, vol.27, 2006, p406

### SUMMARY OF INVENTION

### TECHNICAL PROBLEMS

However, for cell proliferation, it is necessary that the cell culture support has a certain strength or more. Since conventional hydroxyalkyl chitosan gel has weak dynamic strength, it does not cause cell proliferation even when used as a cell culture support. It has been difficult to form a cellular tissue in the gel, and handling at the time of transplantation of the formed cellular tissue and the like has been also difficult. For example, in the case of producing a cellular tissue having a three-dimensional structure in regenerative medicine applications, when cells are cultured in hydroxybutyl chitosan gel, the three-dimensional structure of the gel cannot be remained, and it has been difficult to handle it during production. In addition, three-dimensional shaping of hydroxyalkyl chitosan gel by a three-dimensional shaping apparatus (3D printer) has also been performed. However, since a three-dimensional structure was constructed by laminating very thin cord-like cell-containing gel, it was unable to tolerate its own weight and could not maintain its structure.

An object of the present invention is to improve dynamic characteristics while maintaining temperature responsiveness in a cell culture support containing hydroxyalkyl chitosan.

### SOLUTIONS TO PROBLEMS

That is, the present invention provides a cell culture support having temperature responsiveness, which contains a temperature-responsive hydroxyalkyl chitosan and a water-soluble polymer selected from polyethylene glycol, derivatives thereof, hyaluronic acids, alginic acids and salts thereof.

### ADVANTAGEOUS EFFECTS OF INVENTION

According to the present invention, it is possible to increase and improve dynamic strength of a gel composed of hydroxyalkyl chitosan alone, and to provide a cell culture support which can achieve both temperature responsiveness and excellent dynamic characteristics.

### BRIEF DESCRIPTION OF DRAWINGS

FIG. 1 is a photograph from above of a gel/cell hybrid tissue comprised of hydroxybutyl chitosan, a hyaluronic acid, and human fibroblasts.
FIG. 2 is a micrograph of a cellular tissue slice obtained by removing hydroxybutyl chitosan.
FIG. 3 is a micrograph of a gel/cell hybrid tissue comprised of hydroxybutyl chitosan, a hyaluronic acid and human fibroblasts bioprinted in a grid pattern by a 3D printer.

### DESCRIPTION OF EMBODIMENTS

### <Cell culture support>

The present invention relates to a cell culture support having temperature responsiveness, which contains a temperature-responsive hydroxyalkyl chitosan and a water-soluble polymer selected from polyethylene glycol, derivatives thereof, hyaluronic acids, alginic acids and salts thereof.

The present invention also provides a method for improving dynamic strength of a cell culture support (gel) comprised of hydroxyalkyl chitosan alone, in which a water-soluble polymer selected from polyethylene glycol, derivatives thereof, hyaluronic acids, alginic acids and salts thereof is contained in a temperature-responsive hydroxyalkyl chitosan.

Furthermore, the present invention provides a method for producing a temperature-responsive cell culture support whose strength is improved as compared to the dynamic strength of a cell culture support (gel) comprised of hydroxyalkyl chitosan alone, in which a water-soluble polymer selected from polyethylene glycol, derivatives thereof, hyaluronic acids, alginic acids and salts thereof is contained together with a temperature-responsive hydroxyalkyl chitosan.

### [Temperature-Responsive Hydroxyalkyl Chitosan]

The cell culture support of the present invention contains hydroxyalkyl chitosan (hydroxyalkyl group-introduced chitosan). Examples of the hydroxyalkyl chitosan include hydroxyethyl chitosan, hydroxypropyl chitosan, hydroxyisopropyl chitosan, hydroxybutyl chitosan and hydroxypentyl chitosan, and preferred is hydroxybutyl chitosan.

Hydroxyalkyl chitosan can be produced, for example, by hydroxyalkylating an amino group and/or a hydroxyl group of chitosan with ethylene oxide, propylene oxide, butylene oxide, propylene oxide or the like. Alternatively, chitin may be used as a starting material, hydroxyalkylated, and then deacetylated.

As a hydroxyalkyl chitosan, a temperature-responsive hydroxyalkyl chitosan which is in a fluid state (liquid or sol) at lower critical solution temperature (LCST) or less, and is in a solid (gel) state at LCST or more is used. The LCST of the temperature-responsive hydroxyalkyl chitosan can be adjusted according to its use. For example, the LCST can be raised by reducing the number of introduced hydroxyalkyl groups per monosaccharide, and it can be lowered by increasing the number of introduction. The number of introduced hydroxyalkyl groups per monosaccharide unit of the chitosan molecule is preferably 1.0 to 3.0, more preferably 1.5 to 2.5. The introduction site of the hydroxyalkyl group may be an amino group or a hydroxyl group of chitosan, but it is desirable that at least one is introduced to an amino group per monosaccharide unit.

Although the degree of deacetylation of the hydroxyalkyl chitosan is not particularly limited, it is preferably in the range of 50 to 100% by mol per 1 mol of a pyranose ring, in order to maintain water solubility at a temperature equal to or lower than the LCST.

Although the molecular weight of hydroxyalkyl chitosan is not particularly limited, the number average molecular weight is preferably 3,000 or more, more preferably 10,000 or more, further preferably 100,000 or more, and still more preferably 150,000 or more, in terms of forming an interpenetrating network by entanglement with the water-soluble polymer. The upper limit of the molecular weight of hydroxyalkyl chitosan is not particularly limited as long as temperature responsiveness is exhibited, and those with up to about 1,000,000, preferably up to about 900,000, and more preferably up to about 800,0000 can be used.

In the present specification, the molecular weight of hydroxyalkyl chitosan is obtained by determining quantitatively the reducing end by Shales modified method.

The weight concentration of hydroxyalkyl chitosan in the cell culture support is preferably 50% by weight or less, more preferably 30% by weight or less, further preferably 20% by weight or less, still more preferably 10% by weight or less, in that a change in its shape and/or properties etc. before and after temperature response can be more remarkably exhibited. It is preferably 0.10% by weight or more, more preferably 0.50% by weight or more, further preferably 1.0% by weight or more, and still more preferably 1.5% by weight or more, in that the cell culture support maintains necessary and sufficient dynamic characteristics.

### [Water-Soluble Polymer]

As a water-soluble polymer used in the cell culture support according to the embodiment of the present invention, the one(s) selected from polyethylene glycol, derivatives thereof, hyaluronic acids, alginic acids and salts thereof is used.

Examples of the polyethylene glycol and derivatives thereof include polyethylene glycol active derivatives, preferably multi-arm polyethylene glycol active derivatives, more preferably 2 to 8, and even more preferably 4 to 8 branched multi-arm polyethylene glycol active derivatives. Such multi-arm polyethylene glycol active derivatives are preferably those obtained by mixing (reacting) two kinds of active derivatives having functional groups that can react with each other at the end. As the two kinds of functional groups that can react with each other, a combination of an amino group and an active ester, and a combination of a thiol group and a maleimide group are preferable. As the active ester, those in which N-hydroxysuccinimide and a carboxylic acid are condensed are preferable.

The origin of hyaluronic acid is not particularly limited, and those derived from cockscomb or ligaments, and those produced by lactic acid bacteria or streptococci can be used.

The origin of alginic acid is not particularly limited, and those derived from brown algae or red algae, and those produced by bacteria can be used.

Although the molecular weight of the water-soluble polymer used in the present invention is not particularly limited as long as it is soluble in water, those having a number average molecular weight of about 10,000 to 1,500,000, preferably about 15,000 to 1,200,000 can be used, in terms of forming an interpenetrating network by entanglement with the hydroxyalkyl chitosan. Specifically, as the polyethylene glycol and derivatives thereof, those having a number average molecular weight of about 10,000 or more, preferably about 15,000 or more can be used, and as the alginic acid and hyaluronic acid, those having a number average molecular weight of about 10,000 or more, preferably about 100,000 or more, more preferably about 200,000 or more, further preferably about 300,000 or more, and still more preferably about 400,000 or more, about 1,500,000 or less, and preferably about 1,300,000 or less can be used. The molecular weight of the water-soluble polymer in the present specification is a value determined by gel permeation chromatography (GPC).

The weight concentration of the water-soluble polymer in the cell culture support is preferably 10% by weight or less, more preferably 5.0% by weight or less, and further preferably 3.0% by weight or less, in that a change in its shape and/or properties etc. before and after the temperature response can be more remarkably exhibited. In order to sufficiently improve the dynamic characteristics of the cell culture support, it is preferably 0.01% by weight or more, preferably 0.05% by weight or more, and more preferably 0.1% by weight or more.

It is desirable that the hydroxyalkyl chitosan and the water-soluble polymer in the cell culture support are in a non-crosslinked state, in order to maintain fluidity at LCST or less. By maintaining fluidity at the LCST or less, ejection from a dispensing tip during tissue production by a 3D printer is facilitated.

### [Temperature Responsiveness]

The "temperature responsiveness" as used herein refers to a property that causes a change between a liquid state and a solid state, that is, a sol-gel transition, before the temperature change occurs and after the temperature change occurs, in response to an external temperature change.

A cell culture support has temperature responsiveness, whereby it is possible to disperse cells in a sol state in which fluidity is maintained at lower than the LCST. In addition, the three-dimensional structure can be maintained at the LCST or more during culture to form a three-dimensional cellular tissue. Furthermore, it is possible to remove hydroxyalkyl chitosan by setting to a sol state at lower than the LCST again after three-dimensional cellular tissue formation. The cell culture support in the present invention is desirably in a sol state at room temperature or lower, and a gel state at a cell culture temperature and body temperature, and preferably has an LCST at 4°C to 37°C, and more preferably has an LCST at 25°C to 37°C, in terms of handling and cell culture.

Here, in the present specification, the cell culture support "has temperature responsiveness" means that a ratio (G'(37°C)/G'(4°C)) of a storage elastic modulus at 4°C (G'(4°C)) and a storage elastic modulus at 37°C (G'(37°C)) is greater than 1.0. G'(37°C)/G'(4°C) is preferably 1.5 or more, more preferably 2.0 or more, further preferably 10 or more, and still more preferably 100 or more.

The storage elastic modulus at 4°C is preferably less than 50 Pa, more preferably less than 10 Pa. By having a storage elastic modulus of less than 50 Pa, more preferably less than 10 Pa, suspension of cells and ejection by a 3D printer, and removal after cellular tissue formation are facilitated.

Also, from the viewpoint of exhibiting effects of ease of handling, maintenance of three-dimensional structure and the like, the maximum storage elastic modulus at 25 to 40°C is preferably 100 Pa or more, more preferably 300 Pa or more, and further preferably 1000 Pa or more. Although the upper limit of the maximum storage elastic modulus at 25 to 40°C is not particularly limited, it is generally 50,000 Pa or less. The maximum storage elastic modulus referred to in the present invention refers the maximum storage elastic modulus among the storage elastic moduli in a predetermined temperature range. The storage elastic modulus (G') in the present invention uses a value obtained by placing a sample liquid to be measured in a dynamic viscoelasticity measuring device equipped with a parallel plate at a plate spacing of 0.5 mm and standing for 5 minutes, then measuring at a strain of 1%, a frequency of 1 Hz, and a temperature rise rate of 1.8°C/min. More specifically, the storage elastic modulus at 37°C is preferably 100 Pa or more, more preferably 300 Pa or more, and further preferably 1000 Pa or more. Although the upper limit of the storage elastic modulus at 37°C is not particularly limited, it is generally 50,000 Pa or less.

The weight concentration ratio (hydroxyalkyl chitosan/water-soluble polymer) of the hydroxyalkyl chitosan and the water-soluble polymer in the cell culture support is preferably 1.0 to 100, more preferably 1.0 to 30, and further preferably 1.0 to 25. By setting the weight ratio to 100 or less, more preferably 30 or less, and further preferably 25 or less, the water-soluble polymer is appropriately dispersed with the hydroxyalkyl chitosan, and dynamic characteristics can be improved. When the weight ratio is 0.5 or less, a material comprised of the hydroxyalkyl chitosan, the water-soluble polymer and water may not exhibit temperature responsiveness due to entanglement of the water-soluble polymer, a decrease in contribution of the hydroxyalkyl chitosan, or the like.

In particular, in the case where the water-soluble polymer is polyethylene glycol, when the hydroxyalkyl chitosan/water-soluble polymer is 1.0 or more and 3.5 or less, the effect of improving dynamic characteristics is particularly remarkably exhibited. In particular, in the case where the water-soluble polymer is a hyaluronic acid, when the hydroxyalkyl chitosan/water-soluble polymer is 10 or more and 20 or less, the effect of improving dynamic characteristics is particularly remarkably exhibited. In the case of alginic acid, when the hydroxyalkyl chitosan/water-soluble polymer is 5 or more and 20 or less, the effect of improving dynamic characteristics is particularly remarkably exhibited.

It is preferred that the hydroxyalkyl chitosan and the water-soluble polymer are dispersed and/or dissolved, preferably dissolved in an aqueous solution. Examples of the aqueous solution include water, aqueous buffers (phosphate buffer, TRIS-hydrochloride buffer, acetate buffer, citrate buffer, etc.), physiological saline, aqueous sugar solutions (5% by weight aqueous glucose solution, aqueous dextrose solution, etc.), and cell culture solutions (for example, D-MEM medium, MEM medium, HamF12 medium, HamF10 medium). The concentration of the buffer agent, salt, sugar, and other solutes added to the culture solution in the aqueous solution may be any concentration as long as a hydrogel of hydroxyalkyl chitosan is formed.

### <Cell Culture Support Preparation Kit>

According to the present invention, a cell culture support preparation kit is provided as follows. That is, it includes an aqueous solution A containing a first polyethylene glycol active derivative and an aqueous solution B containing a second polyethylene glycol active derivative capable of reacting with the first polyethylene glycol active derivative, and at least one of the aqueous solution A and the aqueous solution B contains hydroxyalkyl chitosan. As the polyethylene glycol active derivative, the multi-arm polyethylene glycol active derivative described above is preferably used.

Crosslinking is formed between the first and second polyethylene glycol active derivatives by dispersing cells in at least one of the aqueous solution A and the aqueous solution B and then mixing the both. For example, when the aqueous solution A contains an active derivative having an amino group, an aqueous solution B containing an active derivative having an active ester can be used. For example, when the aqueous solution A contains an active derivative having a thiol group, an aqueous solution B containing an active derivative having a maleimide group can be used. In these examples, the ratio of the molar concentration of the polyethylene glycol active derivative in the aqueous solution A to the molar concentration of the polyethylene glycol active derivative in the aqueous solution B (aqueous solution A/aqueous solution B) is preferably 0.5 to 2.0, more preferably 0.9 to 1.1, in order to more efficiently proceed the reaction and sufficiently obtain the effect of improving dynamic characteristics of the cell culture support. Additives such as buffer agents, salts and sugars suitable for cell culture can be added to the aqueous solutions A and B as long as temperature responsiveness is not impaired.

### <Method for Producing Cellular Tissue>

By dispersing cells in the cell culture support of the present invention in a sol state or a liquid state at a temperature below LCST and then heating the cell dispersion to a temperature equal to or higher than the LCST to gelate, a gel/cell hybrid tissue (hereinafter may be referred to as simply "hybrid tissue") can be produced.

In preparing the hybrid tissue, first, cells are dispersed in the cell culture support of the present invention in a sol state or a liquid state at a temperature below the LCST. Hereinafter, a cell culture support in a sol state or a liquid state in which cells are dispersed is referred to as a "liquid cell composition". The cell concentration at this time is preferably 1.0 × 10⁶ to 1.0 × 10⁸ cells/ml, more preferably 3.0 × 10⁷ to 1.0 × 10⁸ cells/ml.

A liquid cell composition can be processed into an arbitrary shape by using a substrate for cell culture having the arbitrary shape. For example, by casting a liquid cell composition onto a substrate for cell culture with a flat plate, the liquid cell composition can be gelled into a sheet by heating to a temperature equal to or higher than the LCST. After pouring the liquid cell composition into a substrate for cell culture which is a three-dimensional template, the liquid cell composition can be sterically gelled by heating to a temperature equal to or higher than the LCST.

As the substrate for cell culture, for example, those formed by plastic having excellent moldability such as polystyrene, polypropylene, polyolefin, polyethylene, polyvinyl chloride, polylactic acid, polyester, polyvinyl alcohol, cellulose, polyacrylate, polysulfone, polycarbonate, polyurethane and polyimide, or inorganic materials such as glass, gold, platinum, copper, titanium, tantalum, zirconia, silica, alumina, hydroxyapatite, calcium phosphate and magnesium phosphate can be used.

A dot pattern can be formed by spotting the liquid cell composition on the substrate for cell culture using a pipette, spotter, arrayer, or the like. When a stamp method, a stencil method, a spray method or the like is used, it is also possible to form a more precise pattern. Such a pattern may be regular or irregular.

Although the temperature at which the shape is fixed is not particularly limited as long as it is equal to or higher than the LCST of the cell culture support, it is preferably 40°C or less, further preferably 37°C or less, in order not to impair the cells. Although the heating time for gelling, that is, fixing the shape is not particularly limited, while, for example, it is preferably 10 seconds to 30 minutes, further preferably 30 seconds to 5 minutes.

After immobilizing the shape by gelling, a culture solution kept at a temperature equal to or higher than the LCST is added thereto, and culturing is performed at a temperature suitable for cell culture equal to or higher than the LCST, whereby a hybrid tissue comprised of the cell culture support of the present invention and a cellular tissue retained in the cell culture support.

### <Bio-Ink>

It is also possible to three-dimensionally shape a cell culture support by using a three-dimensional shaping apparatus (for example, 3D printer or bio printer). That is, a gel/cell hybrid tissue can be produced through a step of shaping a cell dispersion obtained by dispersing cells in the cell culture support of the present invention in a sol state or a liquid state at a temperature lower than lower critical solution temperature, into a cell culture support having a predetermined three-dimensional structure, using a 3D printer. Such procedure is generally called bioprinting. In bioprinting, the liquid cell composition can be precisely placed at an arbitrary position according to a computer program. For example, in bioprinting using a dispenser type three-dimensional shaping apparatus, a liquid cell composition (bio-ink) is prepared by mixing the cell culture support of the present invention in a sol state or a liquid state with cells, and ejected via a dispensing tip (for example, syringe, capillary tube, etc.) connected to a reservoir. Then, the ejected bio-ink is instantaneously heated to a temperature equal to or higher than the LCST, thereby gelling the cell culture support. By repeating this operation according to the computer program, it is possible to three-dimensionally shape a hybrid tissue.

In bioprinting, it is possible to eject bio-ink in a repeating pattern. The repeating pattern can take any suitable geometric structure including, for example, circles, squares, rectangles, triangles, polygons, and irregular geometric shapes. The repeating patterns can be stacked in layers, multiple layers are printed contiguously, and a hybrid tissue is formed.

### <Cellular Tissue Substantially Free of Cell culture support>

After the hybrid tissue is obtained, the temperature is lowered below the LCST, and the cell culture support is again solated and removed, whereby a cellular tissue substantially free of the cell culture support can be obtained. Here, "substantially free of the cell culture support" means a state that the cell culture support does not remain at all, or even if it remains, it is 1% by weight or less in the whole cellular tissue. The temperature for solation of the cell culture support is not particularly limited as long as it is LCST or less, and the lower limit temperature is, for example, 4°C. Although the time for removal is not particularly limited, it is preferably set to 30 seconds to 10 minutes.

The cell culture support of the present invention can be used for a part or whole of a scaffolding molded body used for tissue regeneration or transplantation tissue formation of nerve, heart, blood vessel, cartilage, skin, cornea, kidney, liver, hair, myocardium, muscle, tendon and the like. Specific examples of cells that can be cultured using the cell culture support of the present invention include fibroblasts, vascular endothelial cells, chondrocytes, hepatocytes, small intestinal epithelial cells, epidermal keratinocytes, osteoblasts, bone marrow mesenchymal cells and the like, and preferable examples include fibroblasts. Further, specific examples include cells derived from an organism such as hematopoietic stem cells, neural stem cells, mesenchymal stem cells, mesodermal stem cells, ES cells (embryonic stem cells), pluripotent stem cells, CD34 positive cells, immune system cells, hemocyte cells, nerve cells, pancreatic β-cells, cardiomyocytes, chondrocytes, myoblasts, amniotic cells and umbilical cord blood cells, established cells such as NIH3T3 cells, 3T3-L1 cells, 3T3-E1 cells, Hela cells, PC12 cells, P19 cells, CHO (Chinese hamster oocyte) cells, COS cells, HEK cells, Hep-G2 cells, CaCo2 cells, L929 cells, C2C12 cells, Daudi cells, Jurkat cells, KG-1a cells, CTLL-2 cells, NS-1 cells, MOLT-4 cells, HUT78 cells and MT-4 cells, various hybridoma cell lines that are antibody-producing cells, cells in which these cells are genetically modified by a genetic engineering technique, and the like, but the cells are not limited thereto.

The cell culture support of the present invention can be used for regenerating cellular tissues for regenerative medical applications. The objects to be regenerated include all organs and tissues such as blood vessels, heart valves, pericardium, bones, cartilages, nerves, skin, mucosa, ligaments, tendons, muscle, tracheas, esophaguses, intestines, and duras. After achieving a purpose as a medical molded body or medical material, it is also possible to remove hydroxyalkyl chitosan by temperature change. The gel/cell hybrid tissue containing the cell culture support of the present invention and the cellular tissue retained in the cell culture support may be transplanted, for example, directly into a living body for regenerative medical applications. Application of such techniques will enable the development of devices in medical, research and analytical fields for regenerative medicine, tissue engineering, cell culture, biosensor, biochip and drug screening as applications.

By using tissue-derived cells as cells, for example, a muscle tissue when using myoblasts, a cardiac muscle when using cardiomyocytes, and a blood vessel when using vascular endothelial cells can be formed into a tissue with a shape of the cell culture support.

Hereinafter, the present invention will be specifically described with reference to examples, but the present invention should not be construed as being limited to only those examples, and all technical ideas that are conceived by those skilled in the art who contacted the concepts of the present invention and considered as implementable, and specific modes thereof should be understood as being included in the present invention.

### EXAMPLES

The storage elastic modulus in the examples was measured in accordance with the following method.

Viscoelasticity was measured using a rheometer "Physica MCR302" (registered trademark) made by Anton Paar GmbH. Measurement conditions are shown below.
- Plate: parallel plate (φ 25 mm)
- Plate spacing: 0.5 mm
- Strain: 1%
- Frequency: 1 Hz
- Temperature variation range: 4°C to 40°C
- Temperature change rate: 1.8°C/min

### <Test Example 1: Cell-Free Support for Cell Culture>

### [Sample Preparation Using PEG as Water-Soluble Polymer]

Hydroxybutyl chitosan (molecular weight of 200,000) having the number of introduced hydroxybutyl groups per monosaccharide unit of 2.1 and a 4-branched multi-arm polyethylene glycol active derivative (made by NOF Corporation, molecular weight of 20,000) having an amino group introduced at the end were dissolved in water at 4°C to prepare a solution A. Here, the weight concentration of hydroxybutyl chitosan is 2.5% by weight, and the weight concentration of the 4-branched multi-arm polyethylene glycol active derivative is 0, 0.75, 1.0, 1.5, 2.0, 2.5, 5.0 or 10% by weight.

Separately, hydroxybutyl chitosan (molecular weight of 200,000) having the number of introduced hydroxybutyl groups per monosaccharide unit of 2.1 and a 4-branched multi-arm polyethylene glycol active derivative (made by NOF Corporation, molecular weight of 20,000) having an N-hydroxysuccinimide activated ester introduced at the end were dissolved in water at 4°C to prepare a solution B. Here, the weight concentration of hydroxybutyl chitosan is 2.5% by weight, and the weight concentration of the 4-branched multi-arm polyethylene glycol active derivative is 0, 0.75, 1.0, 1.5, 2.0, 2.5, 5.0 or 10% by weight.

The solution A (200 µL) and the solution B (200 µL) were mixed at 4°C to obtain solutions of 2.5% by weight of hydroxybutyl chitosan, and 0.75, 1.0, 1.5, 2.0, 2.5, 5.0 or 10% by weight of 4-branched multi-arm polyethylene glycol active derivative. The reaction of the amino group and the activated ester was completed by allowing the mixture to stand at 37°C overnight.

### [Sample Preparation Using Hyaluronic Acid or Alginic Acid as Water-Soluble Polymer]

Hydroxybutyl chitosan (molecular weight of 200,000) having the number of introduced hydroxybutyl groups per monosaccharide unit of 1.9 and hyaluronic acid (molecular weight of 1,000,000) or alginic acid (molecular weight of 500,000) were dissolved in water at 4°C to obtain a solution. Here, the weight concentration of hydroxybutyl chitosan is 2.5 or 5.0% by weight, the weight concentration of hyaluronic acid is 0.25 or 0.5% by weight, and the weight concentration of alginic acid is 0.25, 0.5, 0.75, 1 or 2% by weight.

The elastic modulus of each of the support samples for cell culture prepared in Test Example 1 at 37°C and 4°C and the ratio thereof are shown in Table 1.

**[Table 1]**

| Sample number | HBC | Water-soluble polymer | | | HBC/Water-soluble polymer (Weight concentration ratio) | Elastic modulus | | | Temperature responsiveness |
|---|---|---|---|---|---|---|---|---|---|
| | Concentration (% by weight) | Type | Molecular weight | Concentration (% by weight) | | G' (37°C) (Pa) | G'(4°C) (Pa) | G' (37°C)/G' (4°C) | |
| 1 | 2.5 | - | | 0 | - | 8.6 | 0.31 | 28 | + |
| 2 | 2.5 | PEG | 20,000 | 0.75 | 3.3 | 790 | 0.42 | 1881 | + |
| 3 | 2.5 | PEG | | 1.0 | 2.5 | 155 | 0.84 | 185 | + |
| 4 | 2.5 | PEG | | 1.5 | 1.7 | 113 | 19 | 5.9 | + |
| 5 | 2.5 | PEG | | 2.0 | 1.25 | 237.5 | 52 | 4.6 | + |
| 6 | 2.5 | PEG | | 2.5 | 1.0 | 231 | 48 | 4.8 | + |
| 7 | 2.5 | PEG | | 5.0 | 0.5 | 554 | 301 | 1.8 | + |
| 8 | 2.5 | PEG | | 10 | 0.25 | 2026 | 1382 | 1.5 | + |
| 9 | 2.5 | - | | 0 | - | 8.6 | 3.6 | 2.4 | + |
| 10 | 2.5 | Hyaluronic acid | 1,000,000 | 0.25 | 10 | 238 | 9.8 | 24.3 | + |
| 11 | 2.5 | Hyaluronic acid | | 0.5 | 5.0 | 81 | 18 | 4.5 | + |
| 12 | 5 | Hyaluronic acid | | 0 | - | 653 | 12 | 54 | + |
| 13 | 5 | Hyaluronic acid | | 0.25 | 20 | 1618 | 5.6 | 289 | + |
| 14 | 2.5 | Alginic acid | 500,000 | 0.5 | 5 | 32 | 1.3 | 25 | + |
| 15 | 2.5 | Alginic acid | | 0.75 | 3.3 | 108 | 3.4 | 32 | + |
| 16 | 2.5 | Alginic acid | | 1 | 2.5 | 13 | 4.5 | 2.9 | + |
| 17 | 2.5 | Alginic acid | | 2 | 1.25 | 86 | 15 | 5.7 | + |
| 18 | 5 | Alginic acid | | 0.25 | 20 | 1120 | 4.7 | 238 | + |
| 19 | 5 | Alginic acid | | 0.5 | 10 | 2150 | 8.4 | 256 | + |
| 20 | 5 | Alginic acid | | 0.75 | 6.7 | 2040 | 11 | 186 | + |
| 21 | 5 | Alginic acid | | 2 | 2.5 | 825 | 75.3 | 11 | + |

| | | | | | | | | | |
|---|---|---|---|---|---|---|---|---|---|
| *HBC = Hydroxybutyl chitosan * The number of introduced hydroxyalkyl groups of HBC of Sample numbers 1 to 8 is 2.1 * The number of introduced hydroxyalkyl groups of HBC of Sample numbers 9 to 21 is 1.9 | | | | | | | | | |

### <Test Example 2: Support for Cell Culture Containing Cells>

### [Sample Preparation Using PEG as Water-Soluble Polymer]

Hydroxybutyl chitosan (molecular weight of 200,000) having the number of introduced hydroxybutyl groups per monosaccharide unit of 1.8 and a 4-branched multi-arm polyethylene glycol active derivative (made by NOF Corporation, molecular weight of 20,000) having a thiol group introduced at the end were dissolved in a phosphate buffer at 4°C to prepare a solution A. Furthermore, human fibroblasts were dispersed in the solution A. Here, the weight concentration of hydroxybutyl chitosan is 2.5% by weight, and the weight concentration of the 4-branched multi-arm polyethylene glycol active derivative is 0.75% by weight.

Separately, hydroxybutyl chitosan (molecular weight of 200,000) having the number of introduced hydroxybutyl groups per monosaccharide unit of 1.8 and a 4-branched multi-arm polyethylene glycol active derivative (made by NOF Corporation, molecular weight of 20,000) having a maleimide group introduced at the end were dissolved in a phosphate buffer at 4°C to prepare a solution B. Here, the weight concentration of hydroxybutyl chitosan is 2.5% by weight, and the weight concentration of the 4-branched multi-arm polyethylene glycol active derivative is 0.75% by weight.

The solution A (200 µL) and the solution B (200 µL) were mixed at 4°C to obtain cell dispersions of 2.5% by weight of hydroxybutyl chitosan, and 0, 0.75% by weight 4-branched multi-arm polyethylene glycol active derivative (PEG), with a cell concentration of 5.0 × 10⁷ cells/mL. The reaction of the thiol group and the maleimide group was completed by culturing in a culture solution at 37°C overnight. The elastic modulus of the obtained composition was measured while raising the temperature from 4°C to 40°C at 1.8°C/min. The storage elastic modulus (G') at 4°C, 37°C and the ratio thereof are shown in Table 2.

### [Sample Preparation Using Hyaluronic Acid or Alginic Acid as Water-Soluble Polymer]

Hydroxybutyl chitosan (molecular weight of 200,000) having the number of introduced hydroxybutyl groups per monosaccharide unit of 1.8 and hyaluronic acid (molecular weight of 1,000,000), alginic acid (molecular weight of 500,000) were dissolved in a phosphate buffer at 4°C to obtain a solution. Human fibroblasts were dispersed in this solution to obtain a cell dispersion. Here, the weight concentration of hydroxybutyl chitosan is 2.5% by weight, the weight concentration of hyaluronic acid is 0, 0.125 or 0.25% by weight, the concentration of alginic acid is 0.25 or 0.5% by weight, and the cell concentration is 5.0 × 10⁷ cells/mL.

The elastic modulus of each of the support samples for cell culture prepared in Test Example 2 at 37°C and 4°C and the ratio thereof are shown in Table 2.

**[Table 2]**

| Sample number | HBC | Water-soluble polymer | | | HBC/Water-soluble polymer (Weight concentration ratio) | Human fibroblasts | Elastic modulus | | | Temperature responsiveness |
|---|---|---|---|---|---|---|---|---|---|---|
| | Concentration (% by weight) | Type | Molecular weight | Concentration (% by Weight) | | Concentration (cells/mL) | G' (37°C) (Pa) | G' (4°C) (Pa) | G' (37°C)/G' (4°C) | |
| 22 | 2.5 | - | - | 0 | - | 5.0 × 10⁷ | 84 | 2.1 | 39.8 | + |
| 23 | 2.5 | Hyaluronic acid | 1,000,000 | 0.125 | 20 | 5.0 × 10⁷ | 145 | 2 | 72.5 | + |
| 24 | 2.5 | Hyaluronic acid | | 0.25 | 10 | 5.0 × 10⁷ | 634 | 6.8 | 93.2 | + |
| 25 | 2.5 | Alginic acid | 500,000 | 0.25 | 10 | 5.0 × 10⁷ | 194 | 2.8 | 69.2 | + |
| 26 | 2.5 | Alginic acid | | 0.5 | 5.0 | 5.0 × 10⁷ | 147 | 4.8 | 30.5 | + |
| 27 | 2.5 | PEG | 20,000 | 0.75 | 3.3 | 5.0 × 10⁷ | 1171 | 38 | 30.8 | + |

| | | | | | | | | | | |
|---|---|---|---|---|---|---|---|---|---|---|
| * HBC = Hydroxybutyl chitosan * The number of introduced hydroxyalkyl groups of HBC of Sample numbers 22 to 27 is 1.8 | | | | | | | | | | |

### <Production Example 1: Preparation of Gel/Cell Hybrid Tissue>

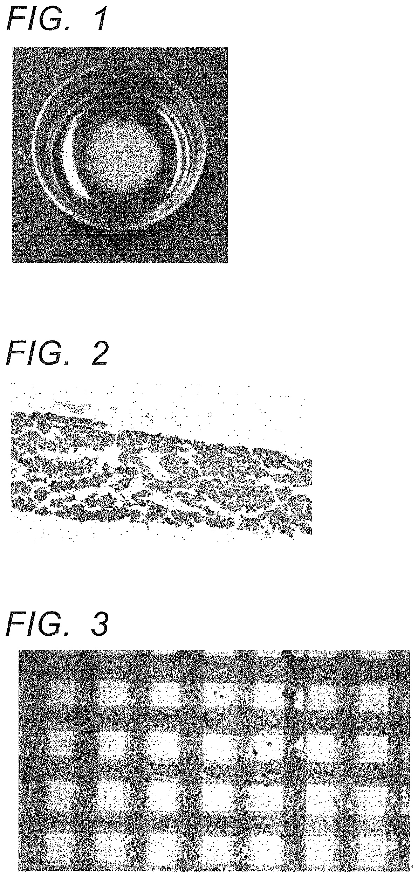

Hydroxybutyl chitosan (molecular weight of 800,000) having the number of introduced hydroxybutyl groups per monosaccharide unit of 1.8 and hyaluronic acid (molecular weight of 1,000,000) were dissolved in a phosphate buffer at 4°C to obtain a solution. Human fibroblasts were dispersed in this solution to obtain a cell dispersion. Here, the weight concentration of hydroxybutyl chitosan is 2.5% by weight, the weight concentration of hyaluronic acid is 0.125% by weight, and the cell concentration is 1.0 × 10⁸ cells/mL. The dispersion was shaped into a circle on a polystyrene dish for cell culture with a diameter of 35 mm and allowed to stand at 37°C for 5 minutes to gelate the dispersion. After adding 37°C cell culture medium (Dulbecco's modified Eagle's medium (DMEM medium) containing 10% fetal bovine serum), the cells were cultured overnight at 37°C to obtain a gel/cell hybrid tissue (FIG. 1).

### <Production Example 2: Preparation of Cellular Tissue Substantially Free of Cell Culture Support>

Hydroxybutyl chitosan (molecular weight of 800,000) having the number of introduced hydroxybutyl groups per monosaccharide unit of 1.8 and hyaluronic acid (molecular weight of 1,000,000) were dissolved in a phosphate buffer at 4°C to obtain a solution. Human fibroblasts were dispersed in this solution to obtain a cell dispersion. Here, the weight concentration of hydroxybutyl chitosan is 2.5% by weight, the weight concentration of hyaluronic acid is 0.125% by weight, and the cell concentration is 1.0 × 10⁸ cells/mL. The dispersion was shaped into a circle on a polystyrene dish for cell culture with a diameter of 35 mm and allowed to stand at 37°C for 5 minutes to gelate the dispersion. After adding 37°C cell culture medium (Dulbecco's modified Eagle's medium containing 10% fetal bovine serum, 2 mL), the cells were cultured overnight at 37°C. After culturing, the culture was cooled at 4°C for 10 minutes to solate the cell culture support, and the medium containing the support was removed by suction. After adding a fresh medium, the cells were further cultured overnight to obtain a cellular tissue substantially free of the cell culture support (FIG. 2).

### <Production Example 3: Preparation of Gel/Cell Hybrid Tissue Using 3D Printer>

Hydroxybutyl chitosan (molecular weight of 800,000) having the number of introduced hydroxybutyl groups per monosaccharide unit of 1.8 and hyaluronic acid (molecular weight of 1,000,000) were dissolved in a phosphate buffer at 4°C to obtain a solution. Human fibroblasts were dispersed in this solution to obtain a cell dispersion. Here, the weight concentration of hydroxybutyl chitosan is 2.5% by weight, the weight concentration of hyaluronic acid is 0.125% by weight, and the cell concentration is 5.0 × 10⁷ cells/mL. The dispersion cooled to 10°C was printed in a grid pattern on a glass bottom dish for cell culture with a diameter of 35 mm kept at 37°C with a 3D printer (SHOTMASTER200DS made by Musashi Engineering, Inc.). After adding a cell culture medium (Dulbecco's modified Eagle's medium containing 10% fetal bovine serum), the cells were cultured overnight at 37°C to obtain a gel/cell hybrid tissue (FIG. 3).

### INDUSTRIAL APPLICABILITY

The present invention can be used as a cell culture support capable of achieving both temperature responsiveness and dynamic characteristics at the same time, and a medical material using the same.

## Claims

1. A cell culture support having temperature responsiveness, comprising a temperature-responsive hydroxyalkyl chitosan and a water-soluble polymer selected from polyethylene glycol, derivatives thereof, hyaluronic acids, alginic acids and salts thereof.

2. A cell culture support of Claim 1, wherein a number average molecular weight of the water-soluble polymer is 20,000 or more.

3. A cell culture support of Claim 1 or Claim 2, wherein a content of the water-soluble polymer is 0.01% by weight or more and 10% by weight or less.

4. A cell culture support of any one of Claim 1 to Claim 3, having a lower critical solution temperature at 4°C to 37°C.

5. A cell culture support of any one of Claim 1 to Claim 4, having a storage elastic modulus at 4°C of less than 50 Pa, and a maximum storage elastic modulus at 25 to 40°C of 100 Pa or more.

6. A cell culture support of any one of Claim 1 to Claim 5, wherein the hydroxyalkyl chitosan and the water-soluble polymer are in a non-crosslinked state.

7. A cell culture support of any one of Claim 1 to Claim 6, wherein the polyethylene glycol comprises 2 kinds of multi-arm polyethylene glycol active derivatives.

8. Cell culture support preparation kit, comprising an aqueous solution A and an aqueous solution B,
wherein the aqueous solution A is an aqueous solution containing a first polyethylene glycol active derivative,
wherein the aqueous solution B is an aqueous solution containing a second polyethylene glycol active derivative, and
wherein at least one of the aqueous solution A and the aqueous solution B contains hydroxyalkyl chitosan.

9. A production method of a gel/cell hybrid tissue, comprising:
a process for dispersing cells in a cell culture support of any one of Claim 1 to 7 in a sol state or a liquid state at a temperature below lower critical solution temperature, and
a process for heating the obtained cell dispersion to a temperature equal to or higher than the lower critical solution temperature to gelate.

10. A production method of a gel/cell hybrid tissue, comprising:
a process in which a cell dispersion obtained by dispersing cells in a cell culture support of any one of Claim 1 to 7 in a sol state or a liquid state at a temperature lower than lower critical solution temperature is shaped into a cell culture support having a predetermined three-dimensional structure, using a 3D printer.
